# FASCICULE DE BREVET EUROPEEN

(11) **EP 4 232 430 B1**
(45) Date de publication et mention de la délivrance du brevet: **23.10.2024**
(21) Numéro de dépôt: 21806312.1
(22) Date de dépôt: 20.10.2021
(51) Int. Cl.: C07C 319/20, C07C 323/58

(54) **PROCÉDÉ DE FABRICATION DE LA MÉTHIONINE**
VERFAHREN ZUR HERSTELLUNG VON METHIONIN
METHOD FOR PRODUCING METHIONINE

(30) Priorité: 23.10.2020 FR 2010881
(43) Date de publication de la demande: 30.08.2023
(73) Titulaire: Adisseo France S.A.S., 92160 Antony (FR); Centre National de la Recherche Scientifique, 75016 Paris (FR); Université Claude Bernard Lyon 1, 69100 Villeurbanne (FR); Ecole Superieure de Chimie, Physique, Electronique de Lyon, 69100 Villeurbanne (FR)
(72) Inventeur: TAULOU, Félix, 69006 Lyon (FR); MORVAN, Didier, 69440 Mornant (FR); BELLIERE-BACA, Virginie, 69390 Millery (FR); GEANTET, Christophe, 01700 Miribel (FR)
(74) Mandataire: Germain Maureau
(86) Numéro de dépôt international: PCT/FR2021/051839
(87) Numéro de publication internationale: WO 2022/084633

(56) Documents cités:
- EP-A1- 3 632 896
- EP-A1- 3 689 851
- WO-A1-2004/089863
- WO-A1-2007/034066
- WO-A1-2020/161067
- CN-A- 113 336 684
- JP-A- H0 393 757
- US-A1- 2017 275 247

## Description

L'invention porte sur une amélioration d'un procédé de fabrication de la méthionine ou de son analogue sélénié (séléno-méthionine), à partir des précurseurs 2-amino-4-méthylthiobutyronitrile ou 2-hydroxy-4-méthylthiobutyronitrile pour la méthionine, ou du 2-amino-4-méthylsélénobutyronitrile ou 2-hydroxy-4-méthylsélénobutyronitrile pour la séléno-méthionine.

La dimension du marché de la méthionine n'est plus à présenter, notamment en nutrition animale, et ses procédés de fabrication sont toujours le champ de nombreux développements. Les dérivés séléniés de la méthionine sont eux aussi des constituants d'intérêts majeurs en nutrition animale.

La préparation de la méthionine peut être opérée par différents procédés mettant en jeu divers intermédiaires de synthèse, et en particulier le 2-amino-4-méthylthiobutyronitrile (AMTBN), le 2-amino-4-méthylthiobutyramide (AMTBM) et le 2-hydroxy-4-méthylthiobutyronitrile (HMTBN).

Le document WO01/60790A1 décrit une synthèse de la méthionine à partir du 2-hydroxy-4-méthylthiobutyronitrile (HMTBN). Par réaction avec l'ammoniac, l'HMTBN est transformé en AMTBN qui, à son tour, est mis à réagir avec l'acétone en milieu basique pour former l'AMTBM. Une hydrolyse catalytique de l'AMTBM, en présence d'un composé de titane présentant une porosité déterminée, conduit au méthioninate d'ammonium à partir duquel la méthionine est récupérée.

On connait selon le document WO2004/089863A1, un procédé de fabrication du sel d'ammonium de l'HMTBA à partir du précurseur nitrile de l'HMTBA, l'HMTBN, selon lequel l'HMTBN en solution aqueuse, mis en présence d'un catalyseur à base de titane, est converti en une seule étape au sel d'ammonium de l'HMTBA. Cette synthèse conduit aussi à la formation de la méthionine et l'HMTBM, et les rendements annoncés en sel d'ammonium de l'HMTBA sont de l'ordre de 10%. Ils sont trop insuffisants pour envisager une application de ce procédé à l'échelle industrielle.

US2017/275247A1 décrit un procédé de préparation de la méthionine à partir de l'amino-nitrile en une seule étape en présence d'eau et d'un catalyseur à base de cérium ou cérine et de zircone.

JPH0393757A concerne un procédé de préparation de la méthionine notamment, à partir de l'amino-nitrile en une seule étape en présence d'eau et d'un catalyseur à base de zircone.

WO2020/161067A1 divulgue aussi un procédé de préparation de la méthionine à partir de l'amino-nitrile en une seule étape en présence d'eau et d'un catalyseur à base de cérium et de zirconium, voire même qu'à partir de zircone.

La présente invention fournit une alternative aux procédés existants permettant de s'affranchir d'une étape au moins, tout en conduisant à la méthionine ou son dérivé sélénié, en des rendements élevés.

Il a été découvert selon l'invention que les intermédiaires amino-nitrile (AMTBN ou son équivalent sélénié) et hydroxy-nitrile (HMTBN ou son équivalent sélénié) pouvaient être transformés en méthionine (ou en séléno-méthionine) en une seule étape, en présence d'eau et d'un catalyseur et, le cas échéant, d'ammoniac ou d'un sel d'ammonium. L'accessibilité et les performances de cette conversion sont telles que sa transposition à une production industrielle de la méthionine est concevable. Par rapport aux procédés de synthèse connus et leurs améliorations qui restent insuffisamment bénéfiques pour que les procédés industriels classiques soient modifiés, la présente invention représente une réelle avancée. Des rendements significatifs sont obtenus en des temps très courts.

Ainsi, l'invention apporte un procédé de préparation d'un composé de formule (I),
[Chem 1]

CH₃XCH₂CH₂C(NH₂)COOH (I)

où X représente S ou Se,
par conversion catalytique d'un composé de formule (II)
   [Chem 2]

   CH₃XCH₂CH₂C(Y)CN (II)

   où X représente S ou Se et Y représente NH₂ ou OH,
lorsque Y représente NH₂, la conversion étant effectuée en présence d'eau et d'au moins un catalyseur comprenant au moins de l'alumine, du dioxyde de titane ou un mélange de ceux-ci, et le cas échéant de NH₃ ou d'un sel d'ammonium, et
lorsque Y représente OH, la conversion étant effectuée en présence d'eau, d'au moins un catalyseur comprenant au moins de l'alumine, du dioxyde de titane, de la zircone ou un mélange de ceux-ci, et de NH₃ ou d'un sel d'ammonium.

Par l'expression « le cas échéant », on entend selon l'invention que, lorsque le procédé implique le précurseur hydroxy-nitrile qui est le composé de formule (II) dans laquelle Y représente OH, la présence de NH₃ ou d'un sel d'ammonium est nécessaire, alors qu'elle ne l'est pas lorsque le procédé implique le précurseur amino-nitrile qui est le composé de formule Il dans laquelle Y représente NH₂. Cette définition n'exclut toutefois pas la présence de NH₃ lorsque le procédé engage le précurseur amino-nitrile, cette variante constituant une mise en oeuvre particulière de l'invention, décrite plus loin.

Un sel d'ammonium selon l'invention comprend tout sel répondant à la formule (NH₄)ₙA où A est notamment choisi parmi les halogènes, les carbonates, les hydrogénocarbonates, les phosphates, les hydrogénophosphates, les sulfates, les hydrogénosulfates, l'acétate, le citrate, formate, hydroxide et n est un entier variant de 1 à 5. A titre d'illustration, il peut être choisi parmi (NH₄)H₂PO₄, (NH₄)₂HPO₄, (NH₄)₃PO₄, (NH₄)HSO₄, (NH₄)₂SO₄, (NH₄)HCO₃ ou (NH₄)₂CO₃

En présence d'un tel catalyseur ci-dessus, le composé de formule (II) peut être transformé directement en méthionine, alors que les procédés connus à partir du composé amino-nitrile ou du composé hydroxy-nitrile nécessitent de passer par l'intermédiaire amino-amide ou hydroxy-nitrile correspondant, qui est ensuite hydrolysé en méthionine, chacune des étapes recourant à des conditions opératoires différentes.

Dans le présent texte, sauf autrement spécifié, on emploiera indifféremment les termes « composé (II) où Y est NH₂ », « AMTBN » et « amino-nitrile » pour désigner le 2-amino-4-méthylthiobutyronitrile, et par analogie, le 2-amino-4-méthylsélénobutyronitrile. De même, les termes « composé (II) où Y est OH », « HMTBN » et « hydroxy-nitrile » renvoient au 2-hydroxy-4-méthylthiobutyronitrile, et par analogie, au 2-hydroxy-4-méthylsélénobutyronitrile. Par composé (II), on entend toutes ces dénominations et donc ces deux composés, pris ensemble ou isolément.

Par « transformé directement », on doit comprendre que, lorsque le procédé est réalisé à l'échelle industrielle, la transformation peut être conduite dans un seul et même réacteur qui comprend le catalyseur, le réacteur étant alimenté en un mélange eau, composé (II) et optionnellement NH₃ ou sel d'ammonium, ou bien par chacun des réactifs séparément, leur mélange étant réalisé dans le réacteur.

Le terme catalyseur tel qu'employé renvoie généralement à la phase active du catalyseur, sans exclure le fait que le catalyseur peut être dopé et/ou supporté.

Par alumine, dioxyde de titane et zircone, on entend tous les polymorphes, le cas échéant, de l'oxyde d'aluminium Al₂O₃, du dioxyde de titane TiOz et du dioxyde de zirconium ZrOz, respectivement, ces formes étant bien connues de l'homme du métier. Le catalyseur peut aussi être une combinaison par deux, voire par trois, de l'alumine, du dioxyde de titane et de la zircone. Il peut aussi comprendre toute autre entité favorisant sa fonction catalytique.

Les caractéristiques, applications et avantages de l'invention sont ci-après exposés plus en détails, étant entendu que ces caractéristiques peuvent être considérées indépendamment les unes des autres, ou en combinaison, quelle que soit la combinaison.

Selon l'invention, le catalyseur comprend ou consiste en un ou plusieurs composés choisis parmi l'alumine, le dioxyde de titane et la zircone ; il(s) constitue(nt) au moins la phase active du catalyseur, optionnellement le support. Ainsi, si le catalyseur n'est pas en totalité constitué de l'un ou plusieurs desdits oxydes, il peut comprendre tout autre composé n'affectant pas les performances du catalyseur, voire les renforçant. Dans une variante de l'invention, le catalyseur consiste en l'un desdits oxydes.

Le catalyseur peut être dopé et/ou supporté. Il peut être dopé par tout élément ou composé classiquement utilisé et bien connu de l'homme du métier. A titre d'illustration, le dopage du catalyseur peut être réalisé par un ou plusieurs des éléments et composés choisis parmi les métaux alcalins, les métaux alcalino-terreux, le lanthane et tout composé des éléments précités. Les éléments suivants K, Cs, Sr, Ba et La sont à privilégier. Si le catalyseur ne consiste pas uniquement en alumine, dioxyde de titane et/ou zircone, il peut aussi être supporté par tout autre composé classiquement utilisé et bien connu de l'homme du métier, et en particulier la silice et les silicoaluminates.

Selon l'invention, l'ensemble des catalyseurs solides mentionnés ci-dessus peuvent être sous forme de poudre ou préférentiellement sous forme de billes, extrudés, tablettes, trilobes ou toute autre forme lui permettant d'être utilisé dans un réacteur, de préférence un réacteur en lit fixe ou autres ou en mode batch dans un réacteur ouvert ou sous pression.

Ledit catalyseur a avantageusement une surface spécifique d'au moins 10 m²/g. En-dessous de cette limite, les performances du catalyseur chutent rapidement avec notamment un déclin de la sélectivité en méthionine au profit de celle en AMBTM ou HMTBM selon le composé (II), et une diminution de la conversion du composé (II). Cette observation s'applique à l'équivalent sélénié. La limite supérieure de la surface spécifique n'est pas déterminante dans le cadre de l'invention, celle-ci étant imposée par les phases actives disponibles dans le commerce. Les valeurs de surface spécifique indiquées dans le présent texte sont déterminées par la méthode la plus courante soit la physisorption d'azote et calculées par la méthode BET.

Dans une mise en oeuvre préférée du procédé de l'invention, le catalyseur est présent en une concentration massique de 0,1% à 200% par rapport à la masse du composé (II), de préférence de 0,5% à 100% et mieux encore de 1% à 50%.

Selon l'invention, des équipements différents peuvent être envisagés pour réaliser la réaction en batch ou continu : le catalyseur solide dopé ou non peut être immobilisé dans un réacteur sous forme de grains ou d'extrudés ou tout autre forme ou supporté sur une mousse métallique. Le réacteur associé à ce type de catalyseur est préférentiellement un lit fixe tubulaire ou multitubulaire, opéré en mode ruisselant ou noyé isotherme ou adiabatique, ou un réacteur échangeur enduit de catalyseur.

La conversion de l'AMTBN ou de l'HMTBN dans le cadre de l'invention est avantageusement effectuée à une température allant de 20°C à 200°C, voire de 50°C à 150°C, et mieux encore de 80°C à 110°C. Il a été constaté, sur une période de réaction allant de l'ordre de 10 minutes à 3 heures, qu'à une température inférieure à 20°C, la réaction est fortement ralentie, et qu'à compter de 110°C, plus la température augmente, plus la sélectivité en dinitrile et polypeptide de la méthionine s'accroît au détriment de celle en méthionine. Sur la plage de 80°C à 110°C, on observe que la sélectivité en méthionine est élevée.

L'AMTBN ou l'HMTBN est généralement en solution aqueuse. Celle-ci peut avoir été préparée en vue de la mise en oeuvre du procédé, ou provenir d'un milieu réactionnel dans lequel l'AMTBN ou l'HMTBN a été produit, respectivement. Dans ce cas, l'AMTBN ou l'HMTBN peut ne pas être pur et comporter des traces, voire des quantités plus grandes mais restant négligeables en ce qu'elles n'agissent pas défavorablement sur la conversion de l'AMTBN ou l'HMTBN selon l'invention.

La concentration de l'AMTBN ou celle de l'HMTBN, selon la transformation mise en oeuvre, peut avoir une influence sur les performances du procédé et notamment lorsqu'elle est trop élevée. Ainsi, selon une variante de l'invention, l'AMTBN est en solution aqueuse en une concentration allant de 0,01 M à 10 M, de préférence de 0,05 M à 1 M, et mieux encore de 0,2 M à 0,4 M. On a relevé qu'au-delà de 1 M, voire même de 0,8 M, si la conversion en AMTBN reste forte, la sélectivité en méthionine diminue alors que celle en AMTBM, en dinitrile et même en polypeptide, respectivement, s'élève.

Lorsque la fabrication de méthionine selon l'invention implique l'HMTBN, il convient d'ajouter de l'ammoniac au milieu réactionnel. Il est de préférence présent en une teneur variant de 1 équivalent à 50 équivalents par rapport à l'HMTBN en ammoniac. L'ammoniac peut être apporté au milieu par toute technique, mais avantageusement, il est approvisionné sous forme d'un bullage en continu.

Il a été observé que la présence d'ammoniac dans la solution d'AMTBM avant sa conversion, améliore significativement la sélectivité en méthionine tout en diminuant la sélectivité en dinitrile croissante au cours du temps en l'absence d'ammoniac. L'invention concerne ainsi une mise en oeuvre avantageuse du procédé décrit ci-dessus dans lequel l'AMTBN est mis en présence d'ammoniac avant d'être mis en contact avec le catalyseur, voire pendant la conversion catalytique au sein du réacteur. De préférence, de l'ammoniac est apporté dans la solution d'AMTBN par bullage, éventuellement à l'aide d'un gaz porteur inerte, tel que l'azote.

L'invention concerne aussi la mise en oeuvre du procédé de l'invention en continu, et avantageusement en présence d'ammoniac, et mieux encore d'un bullage d'ammoniac, dans la solution d'AMTBN, avant sa conversion. Selon cette variante, le procédé est conduit sous pression de 1 à 20 bar, de préférence de 2 et 10 bar. Ainsi, l'invention fournit un dispositif comprenant une cuve pour la solution d'AMTBN et dans laquelle un bullage d'un mélange d'ammoniac et d'azote est prévu. La solution d'AMTBN est pompée vers un réacteur en inox qui comprend le catalyseur et qui est chauffé au moyen d'un manchon à une température de 80 à 100°C. Le milieu réactionnel est soutiré vers un séparateur gaz/liquide duquel l'ammoniac sera retiré et depuis lequel le liquide sera traité pour récupérer la méthionine. La solution est alors évaporée jusqu'à obtenir un solide, puis le solide est recristallisé dans un mélange eau/éthanol (1/6) à 60°C. La méthionine ainsi obtenue sous forme d'un solide blanc est lavée, filtrée puis séchée. Ce procédé en continu décrit pour l'obtention de méthionine à partir d'AMTBN s'applique à l'identique à l'obtention de méthionine à partir d'HMTBN, l'apport en ammoniac étant toutefois indispensable.

L'invention et ses avantages sont illustrés dans les exemples ci-après.

### Exemple 1 : Préparation de méthionine à partir d'AMTBN en présence de TiO₂, selon l'invention

La réaction d'hydrolyse de l'AMTBN et les conditions dans lesquelles elle est conduite sont décrites dans le schéma ci-dessous.

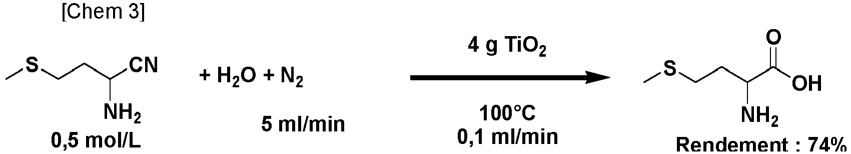

Dans un flacon à vis de 1 litre, on introduit 65 g d'AMTBN avec 1000 ml d'H₂O. La solution est agitée à température ambiante avec un flux d'azote (5 ml/min), la solution est injectée dans un réacteur tubulaire chauffé à 100°C avec un débit de 0,1 ml/min (temps de contact 10 minutes) et contenant 4 grammes de TiOz (anatase, 150 m²/g, Norpro, ST 61120). La réaction est suivie sur 48 heures par RMN du proton.

La conversion de l'AMTBN est supérieure à 90%, le rendement en méthionine est en moyenne de 74% avec une sélectivité moyenne de 81% et le rendement en dinitrile est en moyenne de 11% avec une sélectivité moyenne 12%.

### Exemple 2 : Préparation de méthionine à partir d'AMTBN en présence de TiO₂ et d'ammoniac, selon l'invention - Influence de la surface spécifique de TiO₂

La réaction d'hydrolyse de l'AMTBN et les conditions dans lesquelles elle est conduite sont décrites dans le schéma ci-dessous.

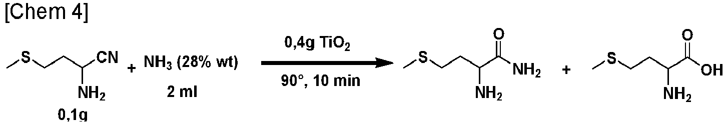

### 2.1 TiOz (sous forme d'anatase) d'une BET de 90 m²/g

Dans un flacon de 10 ml, 0,4 g de TiOz (sous forme d'anatase) (90 m²/g), puis 0,1 g d'AMTBN (98%) avec 2 mL d'une solution d'ammoniaque à 28% en poids ont été introduits. La solution a été chauffée à 90°C pendant 10 minutes au bout desquelles la solution a été filtrée et analysé par RMN du proton.

Les rendements sont en méthionine de 93%, en AMTBM de 1% et en dinitrile de 6%.

### 2.2 TiOz (sous forme d'anatase) d'une BET de 275 m²/g

Dans un flacon de 10 ml, 0,4 g de TiO₂ (sous forme d'anatase) (275 m²/g), puis 0,1 g d'AMTBN (98%) avec 2 mL d'une solution d'ammoniaque à 28% en poids ont été introduits. La solution a été chauffée à 90°C pendant 10 minutes au bout desquelles la solution a été filtrée et analysé par RMN du proton.

Les rendements sont en méthionine de 95%, en AMTBM de 1% et en dinitrile de 4%.

On préférera un catalyseur TiOz ayant une BET d'au moins 90%.

### Exemple 3 : Préparation de la méthionine à partir d'AMTBN, en présence de dioxyde de titane dopé et d'ammoniac, selon l'invention

Cet exemple porte l'utilisation de TiOz dopé au césium et au strontium, respectivement. Le dopage a été réalisé par une méthode d'imprégnation du TiOz avec de l'hydroxyde de césium ou de l'hydroxyde de strontium, avec une teneur de 4% en masse de césium et de strontium (non métallique).

Une solution d'AMTBN à 0,8 mol/L est mise en contact avec 5 g de l'un ou l'autre des catalyseurs dopés pendant 10 minutes à une température de 100°C.

Les résultats sont présentés dans le tableau 1 ci-dessous.

**[Tableau 1]**

| Dopage | Conversion de l'AMTBN | Sélectivité en Met | Sélectivité en AMTBM | Sélectivité en dinitrile | Sélectivité en autres |
|---|---|---|---|---|---|
| Cs | 95% | 88% | 0% | 7% | 5% |
| Sr | 96% | 80% | 13% | 4% | 3% |

### Exemple 4 : Préparation de la méthionine à partir d'AMTBN en présence de dioxyde de titane et d'ammoniac selon un procédé en continu de l'invention

La réaction d'hydrolyse de l'AMTBN et les conditions dans lesquelles elle est conduite sont décrites dans le schéma ci-dessous.

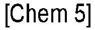

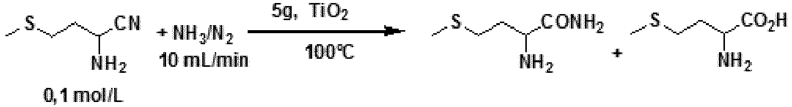

Le catalyseur est un oxyde de titane ayant une surface spécifique de 150 m²/g.

5 g de ce catalyseur sont placés dans le réacteur où une solution aqueuse d'AMTBN à 0,1 mol/L est mise à circuler avec un débit de 0,2 ml/min, et un flux d'ammoniac de 10 ml/min. La température de réaction est 100°C et le temps de contact est de 6 minutes.

Les résultats sont présentés dans le tableau 2 ci-dessous.

**[Tableau 2]**

| Durée du suivi | Conversion de l'AMTBN | Sélectivité en Met | Sélectivité en AMTBM | Sélectivité en dinitrile | Sélectivité en autres |
|---|---|---|---|---|---|
| 1h | 94% | 88% | 5% | 3% | 4% |
| 4h | 96% | 90% | 2% | 4% | 4% |

On observe que le système est stable en conversion et en sélectivité, avec une forte sélectivité en méthionine (90%), une conversion très élevée de l'AMTBN (96%) et une faible sélectivité vers les autres produits. Les rendements en méthionine et en AMTBM sont respectivement de 86% et de 2%.

### Exemple 4 : Préparation de méthionine à partir d'HMTBN, en présence d'un dioxyde de titane et de diammonium hydrogénophosphate, selon l'invention

La réaction d'hydrolyse de l'HMTBN et les conditions dans lesquelles elle est conduite sont décrites dans le schéma ci-dessous.

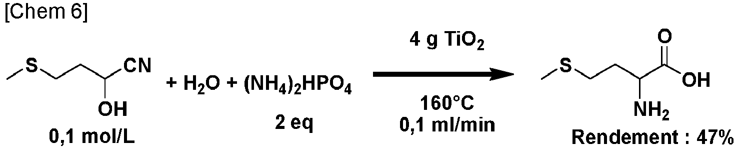

Dans un flacon à vis de 1 litre, on introduit 13,1 g d'HMTBN avec 1000 ml d'H₂O. La solution est agitée à température ambiante avec un flux d'azote, la solution est injectée dans un réacteur tubulaire chauffé à 160°C avec un débit de 0,1 ml/min (temps de contact 10 minutes) et contenant 4 grammes de TiOz (anatase, 150 m²/g, Norpro, ST 61120). La réaction est suivie par HPLC, le rendement en méthionine est de 47%.

### Exemple 5: Préparation de méthionine à partir de HMTBN, en présence d'un dioxyde de titane et d'ammoniac, selon l'invention

La réaction d'hydrolyse de l'HMTBN et les conditions dans lesquelles elle est conduite sont décrites dans le schéma ci-dessous.

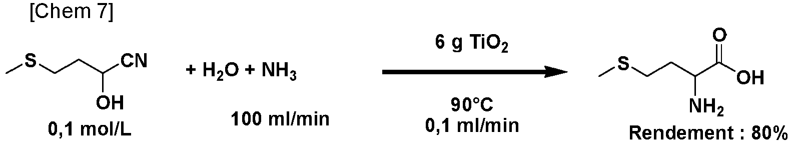

Dans un flacon à vis de 1 litre, on a introduit 13,1 g d'HMTBN avec 1000 ml d'H₂O. La solution est agitée à température ambiante avec un flux d'ammoniac avec un débit de 100 ml/min, la solution est injectée dans un réacteur tubulaire chauffé à 90°C avec un débit de 0,1 ml/min (temps de contact 15 minutes) et contenant 6 grammes de TiOz (anatase, 150 m²/g, Norpro, ST 61120). La réaction est suivie par HPLC, le rendement en méthionine de 80%.

### Exemple 6 : Préparation de méthionine en présence d'un dioxyde de titane, à partir d'HMTBN mais sans source d'ammoniac, selon l'art antérieur

La réaction d'hydrolyse de l'HMTBN et les conditions dans lesquelles elle est conduite sont décrites dans le schéma ci-dessous.

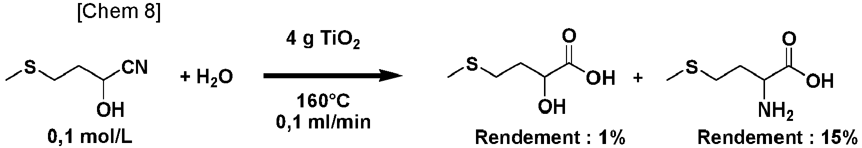

Dans un flacon à vis de 1 litre, on a introduit 13,1 g d'HMTBN avec 1000 ml d'H₂O. La solution est agitée à température ambiante avec un flux d'azote, la solution est injectée dans un réacteur tubulaire chauffé à 160°C avec un débit de 0,1 ml/min (temps de contact 10 minutes) et contenant 4 grammes de TiOz (anatase, 150 m²/g, Norpro, ST 61120). La réaction est suivie par HPLC, le rendement en HMTBA est de 1% et de 15% en méthionine.

La comparaison des résultats des exemples 4 à 5 selon l'invention, avec ceux obtenus dans un procédé conduit sans ammoniac ou sel d'ammonium à l'exemple 6, démontre un gain considérable des performances de la fabrication de la méthionine dans un procédé de l'invention. Le même bénéfice est observé dans la fabrication de la sélénométhionine.

## Revendications

1. Procédé de préparation d'un composé de formule I,
[Chem 1]
CH₃XCH₂CH₂C(NH₂)COOH (I)
où X représente S ou Se,
par conversion catalytique d'un composé de formule Il
[Chem 2]
CH₃XCH₂CH₂C(Y)CN (II)
où X représente S ou Se et Y représente NH₂ ou OH,
**caractérisé en ce que**,
lorsque Y représente NH₂, la conversion est effectuée en présence d'eau et d'au moins un catalyseur comprenant au moins de l'alumine, du dioxyde de titane ou un mélange de ceux-ci, et
lorsque Y représente OH, la conversion est effectuée en présence d'eau, d'au moins un catalyseur comprenant au moins de l'alumine, du dioxyde de titane, de la zircone ou un mélange de ceux-ci, et de NH₃ ou d'un sel d'ammonium.

2. Procédé selon la revendication 1, **caractérisé en ce qu'**il implique le composé de formule Il dans laquelle Y représente OH et ladite conversion est effectuée en présence d'eau, d'au moins un catalyseur comprenant ou consistant en au moins de l'alumine, du dioxyde de titane, de la zircone ou un mélange de ceux-ci, et NH₃ ou un sel d'ammonium.

3. Procédé selon la revendication 1 ou 2, **caractérisé en ce que** ledit catalyseur est dopé, de préférence il est dopé avec un ou plusieurs des éléments et composés choisis parmi les métaux alcalins, les métaux alcalino-terreux, le lanthane et tout composé des éléments précités, et de préférence au moins l'un de K, Cs, Sr, Ba.

4. Procédé selon l'une quelconque des revendications 1 à 3, **caractérisé en ce que** ledit catalyseur a une surface spécifique BET d'au moins 10 m²/g.

5. Procédé selon l'une quelconque des revendications 1 à 4, **caractérisé en ce que** le catalyseur est en une concentration massique de 0,1% à 200% par rapport à la masse du composé (II), de préférence de 0,5% à 100% et mieux encore de 1% à 50%.

6. Procédé selon l'une quelconque des revendications 1 à 5, **caractérisé en ce que** le composé de formule (II) avec Y représente NH₂ est en solution aqueuse en une concentration allant de 0,01 M à 10 M, de préférence de 0,05 M à1 M, et mieux encore de 0,2 à 0,4 M.

7. Procédé selon l'une quelconque des revendications 1 à 6, **caractérisé en ce que** la conversion est effectuée à une température allant de 20°C à 200°C, de préférence de 50°C à 150°C, et mieux encore de 80°C à 110°C.

8. Procédé selon l'une quelconque des revendications 1 à 7, **caractérisé en ce que**, avant la conversion, le composé de formule (II) avec Y représentant NH₂ est mis en présence d'ammoniac.

9. Procédé selon l'une quelconque des revendications 1 à 7, **caractérisé en ce que** le composé de formule (II) avec Y représentant OH, est mis en présence d'un sel d'ammonium choisi parmi (NH₄)H₂PO₄, (NH₄)₂HPO₄, (NH₄)₃PO₄, (NH₄)HSO₄, (NH₄)₂SO₄, (NH₄)HCO₃ ou (NH₄)₂CO₃

10. Procédé selon l'une quelconque des revendications 1 à 9, **caractérisé en ce qu'**il est conduit en continu.

## Patentansprüche

1. Verfahren zum Herstellen einer Verbindung der Formel I,
[Chem 1]
CH₃XCH₂CH₂C(NH₂)COOH (I),
wobei X für S oder Se steht,
durch katalytische Umwandlung einer Verbindung der Formel II
[Chem 2]
CH₃XCH₂CH₂C(Y)CN (II),
wobei X für S oder Se steht und Y für NH₂ oder OH steht,
**dadurch gekennzeichnet, dass**,
wenn Y für NH₂ steht, die Umwandlung in Gegenwart von Wasser und mindestens einem Katalysator, der mindestens Aluminiumoxid, Titandioxid oder eine Mischung davon umfasst, erfolgt, und
wenn Y für OH steht, die Umwandlung in Gegenwart von Wasser, mindestens einem Katalysator, der mindestens Aluminiumoxid, Titandioxid, Zirkoniumoxid oder eine Mischung davon umfasst, und NH₃ oder einem Ammoniumsalz erfolgt.

2. Verfahren nach Anspruch 1, **dadurch gekennzeichnet, dass** es sich um die Verbindung der Formel II handelt, in der Y für OH steht, und die Umwandlung in Gegenwart von Wasser, mindestens einem Katalysator, der mindestens Aluminiumoxid, Titandioxid, Zirkoniumoxid oder eine Mischung davon umfasst oder daraus besteht, und NH₃ oder einem Ammoniumsalz erfolgt.

3. Verfahren nach Anspruch 1 oder 2, **dadurch gekennzeichnet, dass** der Katalysator dotiert ist, vorzugsweise ist er mit einem oder mehreren der Elemente und Verbindungen dotiert, die aus Alkalimetallen, Erdalkalimetallen, Lanthan und jeder Verbindung der oben genannten Elemente und vorzugsweise mindestens einem von K, Cs, Sr, Ba ausgewählt werden.

4. Verfahren nach einem der Ansprüche 1 bis 3, **dadurch gekennzeichnet, dass** der Katalysator eine spezifische BET-Oberfläche von mindestens 10 m²/g aufweist.

5. Verfahren nach einem der Ansprüche 1 bis 4, **dadurch gekennzeichnet, dass** der Katalysator in einer Massenkonzentration von 0,1 % und 200 %, bezogen auf die Masse der Verbindung (II), vorzugsweise von 0,5 % bis 100 % und noch besser von 1 % bis 50 % vorliegt.

6. Verfahren nach einem der Ansprüche 1 bis 5, **dadurch gekennzeichnet, dass** die Verbindung der Formel (II), wobei Y für NH₂ steht, in wässriger Lösung in einer Konzentration im Bereich von 0,01 M bis 10 M, vorzugsweise von 0,05 M bis 1 M und noch besser von 0,2 bis 0,4 M vorliegt.

7. Verfahren nach einem der Ansprüche 1 bis 6, **dadurch gekennzeichnet, dass** die Umwandlung bei einer Temperatur im Bereich von 20 °C bis 200 °C, vorzugsweise von 50 °C bis 150 °C und noch besser von 80 °C bis 110 °C erfolgt.

8. Verfahren nach einem der Ansprüche 1 bis 7, **dadurch gekennzeichnet, dass** die Verbindung der Formel (II), wobei Y für NH₂ steht, vor der Umwandlung mit Ammoniak umgesetzt wird.

9. Verfahren nach einem der Ansprüche 1 bis 7, **dadurch gekennzeichnet, dass** die Verbindung der Formel (II), wobei Y für OH steht, mit einem Ammoniumsalz, das aus (NH₄)H₂PO₄, (NH₄)₂HPO₄, (NH₄)₃PO₄, (NH₄)HSO₄, (NH₄)₂SO₄, (NH₄)HCO₃ oder (NH₄)₂CO₃ ausgewählt wird, umgesetzt wird.

10. Verfahren nach einem der Ansprüche 1 bis 9, **dadurch gekennzeichnet, dass** es kontinuierlich durchgeführt wird.

## Claims

1. A method for preparing a compound of formula I,
[Chem 1]
CH₃XCH₂CH₂C(NH₂)COOH (I)
where X represents S or Se,
by catalytic conversion of a compound of formula II
[Chem 2]
CH₃XCH₂CH₂C(Y)CN (II)
where X represents S or Se and Y represents NH₂ or OH,
**characterized in that**,
when Y represents NH₂, the conversion is performed in the presence of water and of at least one catalyst comprising at least alumina, titanium dioxide or a mixture thereof, and
when Y represents OH, the conversion is performed in the presence of water, of at least one catalyst comprising at least alumina, titanium dioxide, zirconia or a mixture thereof, and of NH₃ or of an ammonium salt.

2. The method according to claim 1, **characterized in that** it involves the compound of formula II in which Y represents OH and said conversion is performed in the presence of water, of at least one catalyst comprising or consisting of at least alumina, titanium dioxide, zirconia or a mixture thereof, and of NH₃ or of an ammonium salt.

3. The method according to claim 1 or 2, **characterized in that** said catalyst is doped, preferably it is doped with one or more of the elements and compounds chosen from alkali metals, alkaline earth metals, lanthanum and any compound of the abovementioned elements, and preferably at least one of K, Cs, Sr, Ba.

4. The method according to any one of claims 1 to 3, **characterized in that** said catalyst has a BET specific surface area of at least 10 m²/g.

5. The method according to any one of claims 1 to 4, **characterized in that** the catalyst is in a mass concentration from 0.1% to 200% relative to the mass of the compound (II), preferably from 0.5% to 100% and better still from 1% to 50%.

6. The method according to any one of claims 1 to 5, **characterized in that** the compound of formula (II) with Y representing NH₂ is in aqueous solution in a concentration ranging from 0.01 M to 10 M, preferably from 0.05 M to 1 M, and better still from 0.2 to 0.4 M.

7. The method according to any one of claims 1 to 6, **characterized in that** the conversion is performed at a temperature ranging from 20°C to 200°C, preferably from 50°C to 150°C, and better still from 80°C to 110°C.

8. The method according to any one of claims 1 to 7, **characterized in that**, before the conversion, the compound of formula (II) with Y representing NH₂ is brought into contact with ammonia.

9. The method according to any one of claims 1 to 7, **characterized in that** the compound of formula (II) with Y representing OH, is brought into contact with an ammonium salt chosen from (NH₄)H₂PO₄, (NH₄)₂HPO₄, (NH₄)₃PO₄, (NH₄)HSO₄, (NH₄)₂SO₄, (NH₄)HCO₃ or (NH₄)₂CO₃.

10. The method according to any one of claims 1 to 9, **characterized in that** it is carried out continuously.
